# EUROPEAN PATENT SPECIFICATION

(11) **EP 2 363 135 B1**
(45) Date of publication and mention of the grant of the patent: **07.05.2014**
(21) Application number: 10382048.6
(22) Date of filing: 01.03.2010
(51) Int. Cl.: A61K 33/00, A61K 33/30, A61Q 17/00, A61K 36/00, A61K 36/28, A61K 36/258, A61K 36/82, A61K 36/886, A61K 36/236

(54) **Anti-jellyfish compositions**
Zusammensetzung gegen Quallen
Compositions contre les méduses

(43) Date of publication of application: 07.09.2011
(73) Proprietor: Antonio Puig, S.A., 08021 Barcelona (ES)
(72) Inventor: Ginestar González, José, 08030, Barcelona (ES); Panyella Costa, David, 08030, Barcelona (ES); Recasens Gracia, Mar, 08030, Barcedlona (ES); Campderrós Serramia, Laia, 08030, Barcelona (ES); Galindo Parres, Silvia, 08030, Barcelona (ES)
(74) Representative: ZBM Patents - Zea, Barlocci & Markvardsen

(56) References cited:
- EP-A2- 0 282 002
- WO-A1-96/19182
- WO-A1-03/096993
- WO-A2-2005/076731
- CN-A- 1 795 840
- US-A1- 2006 083 708

## Description

The present invention relates to the use of compositions for avoiding the sting of venomous organisms from aquatic environments. Particularly, the invention relates to the use of compositions for avoiding the sting of cnidarians and myxozoans.

### BACKGROUND ART

Jellyfish stings are a common occurrence among divers and beach-lovers worldwide with an estimated 150 million envenomations annually. Fatalities and hospitalizations occur frecuently in the Indo-Pacific regions, while fatal stings are unusual in Europe. Despite the lower mortality, jellyfish envenomation is nevertheless an over-all concern in European beaches, specially for children.

In addition to jellyfish, other animals of the same group, the phylum Cnidaria, are toxic to humans to greater or lesser effect. The Cnidaria phylum includes animals like sea anemones, sea wasps, corals and box jellyfish. All of these animals posses a type of venomous cells, called cnidocytes, that they use mainly for capturing prey. These cells are responsible for the stinging of Cnidaria.

Each cnidocyte cell contains a organelle called a cnidocyst, which comprises a bulb-shape capsule containing a coiled hollow thread-like structure attached to it. The externally-oriented side of the cell also has a hair-like trigger called a cnidocil. The cnidocyst capsule stores a large concentration of calcium ions. When the trigger is activated by certain stimuli, the calcium ions are released from the capsule into the cytoplasm of the cnidocyte causing a large concentration gradient of calcium across the cnidocyte plasma membrane. The resulting osmotic pressure causes a rapid influx of water into the cell. This increase in water volume in the cytoplasm forces the coiled cnidocyst to eject rapidly penetrating the target organism, and the hollow thread-like structure is everted into it. This discharge takes no more than a few microseconds, and is said to be one of the most rapid mechanical events found in nature. After penetration, the toxic content of the cnidocyst is injected into the target organism.

In order to regulate discharge, cnidocytes are connected as "batteries", containing several types of cnidocytes connected to supporting cells and neurons. The supporting cells contain chemoreceptors, which, together with the mechanoreceptor on the cnidocyte (cnidocil), allow only the right combination of stimuli to cause discharge, such as prey swimming, and chemicals found in prey cuticle or skin.

Parasitic animals of the group of the myxozoans posses organells that highly resemble cnidocysts. In the case of myxozoans, these organells are named polar capsules and play a major role in the infectious cycle of these aquatic parasites.

Post-stinging remedies such as soothing creams and antidotae are usually applied in the treatment of cnidarian envenomation. However, the effectiveness of these remedies are often unsatisfactory so that the best practice is to avoid contact with these animals. Rash guards and stinger suits are excellent in preventing contact with cnidarians and are recommended for divers in regions where lethal jellyfish are common, but they are not practical for bathers, nor are they completely effective when dealing with myxozoan infection. Therefore, other prevention strategies are desirable to avoid the sting of cnidarians and the infection by myxozoans.

In this direction, Salleo et al (J.exp Biol.,1994, vol. 187, p. 201-206) disclosed that Gd³⁺ and La³⁺ could be useful in preventing stings from harmful Cnidaria. These authors investigated the activation properties of in situ cnidocytes of Pelagia noctiluca (Scyphozoa) by physical contact with a gelatin probe that, besides stimulating the cnidocyte battery, retained the discharged cnidocysts, thereby allowing a quantitative evaluation of the response. In oral arms previously treated with 2 mmol/L La³⁺ or 20 µmol/L Gd³⁺, the discharge was inhibited. This results confirmed the Ca²⁺-dependence of cnidocyte activation and indicated that these cations could be useful to inhibit discharge.

Additionaly, WO 98/53807 discloses compositions comprising an effective amount of antihistamines and/or an effective amount of at least one metal cation for inhibiting cnidocyst or polar capsule discharge. The preferred metal cations for this use include Ca, Mg, Na, Mn, Co, K and Fe. In a further patent application, WO05/076731, the same author discloses compositions comprising an oil in water emulsion containing an inorganic salt, wherein said salt comprises Ca, Mg, Na, K, Zn, Ti, Ga or La cation for the same use. Preferred compositions are those containing Ca, Mg, and La cations. The provided emulsions are to be used in the prevention of cnidocyst discharge.

In a collaborative study, the protective effects of a composition containing Ca and Mg cations formulated in a sunscreen lotion was evaluated against Chrysaora fuscescens and Chiropsalmus quadrumanus jellyfish (Boer Kimball A. et al, Wilderness and Environmental Medicine, 2004, vol. 15, p. 102-108). The study concluded that the jellyfish sting inhibitor does not eliminate the sting from C fuscescens or C quadrumanus jellyfish, but significantly reduces the frequency and severity of stings, thus providing some positive effects.

From what is known in the art, it is derived that further strategies are needed to minimise the effects of cnidarian envenomation and provide practical protection for recreational and professional swimmers. Therefore, the provision of compositions useful for inhibiting the discharge of cnidocysts, thereby avoiding the sting of cnidarians and the infection by myxozoans, is still of great interest in the fields of cosmetics and pharmacy.

### SUMMARY OF THE INVENTION

It has been surprisingly found that compositions comprising plant extracts inhibit the discharge of cnidocysts and polar capsules, thus being useful for avoiding the envenomation caused by the sting of poisonous organisms that contain such cnidocysts. The compositions of the invention have the advantage, with respect to the known treatments against cnidarian envenomation, of containing natural inhibitors of cnydocyst discharge.

In the last years there has been a strong interest in plant extracts and phytochemicals from the basis of their purported health benefits - not only from the functional food and supplement industries but also from cosmetic and pharmaceutical companies. The recovery of the knowledge and practices associated with these plant resources are part of an important strategy linked to the conservation of biodiversity, discovery of new active compounds, and the bettering of the quality of life of poor rural communities.

The present inventors have found a new practical application for plant extracts.

Thus, a first aspect of the present invention relates to the use of a topical composition comprising an effective amount of at least one plant extract, together with appropriate topical cosmetically, and/or pharmaceutically acceptable carriers and/or excipients, for avoiding the sting of poisonous organisms that contain cnidocysts.

As mentioned above, venomous organisms of the Cnidaria and Myxozoa groups are of special concern for the public health in coastal areas. By inhibiting the discharge of cnidocysts and polar capsules, the compositions of the invention avoid the sting of those organisms, thus preventing infection and toxic effects.

Another aspect of the invention is a method for avoiding the sting of an organism selected from the group consisting of Cnidaria and Myxozoa comprising the topical administration of a composition comprising an effective amount of at least one plant extract, together with appropiate topical pharmaceutically and/or cosmetically acceptable excipients or carriers, prior to contact with said organism.

The compositions according to the present invention have the general advantage of containing natural ingredients. Plant extracts have a large history of medicinal and cosmetical use, specially in oriental regions, and have become increasingly popular in western countries due to their beneficial properties. The compositions of the invention exhibit additional advantages that are related to the different plant extract components. For instance, tea extract and oat extract contain flavones and polyphenols, which are known to be strong antioxidants and have been reported to exhibit anti-cancer properties. Additionally, oat extract is often used in topical preparations because of its anti-irritant and anti-itching effects. Olive leaf extract contains another potent antioxidant, hydroxytyrosol, while liquorice extract contains a highly antioxidant and anti-inflammatorial compound called glycyrrhizate.

As mentioned above, some metal cations, such as lanthanum, gadolinium, zinc, calcium, sodium, potassium, magnesium, and titanium, have been described as exhibiting an inhibitory effect for the discharche of cnidarian cnidocysts. Since cnidocyst discharge is a Ca²⁺-dependent process, the inhibitory effect is attributed to their Ca²⁺ channel blocking activity (see Salleo et al, supra).

It has been now surprisingly found that the combination of these previously described substances with new inhibitory agents improves the effects of anti-cnidarian compositions by having a synergistic effect, without having antagonistic effects. As shown in the examples below, the combination of a plant extract with certain metal cations shows sinergistic effects in the inhibition of the discharge of cnidocysts. Therefore, these compositions, which also form part of the invention, have the advantage that they exhibit a better performance in preventing the sting of cnidarians, and the infection by myxozoans.

Finally, another aspect of the present invention relates to the use of a combination for simultaneous or sequential administration of at least one plant extract, and an external source of a cation metal selected from the group consisting of lanthanum, gadolinium, zinc, calcium, sodium, potassium, magnesium, and titanium, for avoiding the sting of poisonous organisms that contain cnidocysts.

It also forms part of the invention the use of a topical composition comprising the previous combination together with appropriate topical pharmaceutically or cosmetically acceptable excipients or carriers for avoiding the sting of poisonous organisms that contain cnidocysts.

### DETAILED DESCRIPTION OF THE INVENTION

Cnidaria is a phylum containing over 9000 species of animals found exclusively in aquatic and mostly marine environments. Some cnidarians are well known by their common name, for instance, jellyfish, sea anemones, sea wasps, corals and box jellyfish. Their distinguishing feature is cnidocytes, specialized stinging cells they use mainly for capturing prey.

A cnidocyte (also called nematocyte or cnidoblast) is a type of venomous cell unique to the phylum Cnidaria. These cells contain a stinging structure called cnidocysts, as well as an externally-orientated hair-like trigger called a cnidocil.

The term "cnidocyst" (also called cnida or nematocyst) is defined as the intracellular, stinging, capsule-like structure characteristic of all cnidarians. When triggered, the cnidocyst is rapidly ejected penetrating a target organism and everting a hollow thread-like structure which injects neurotoxins into it. This mechanism is responsible for the sting delivered by certain cnidarias, such as jellyfish.

Myxozoans are parasitic animals of aquatic environments. Infection occurs through valved spores that contain one or two sporoblast cells and one or more polar capsules that contain filaments which anchor the spore to its host. The sporoblasts are then released as a motile form, called an amoebula, which penetrates the host tissues. Phylogenetical studies, as well as the great similarity between myxozoan polar capsules and cnidarian cnidocysts, have driven many experts to consider myxozoans as severely modified members of the phylum Cnidaria. In the sense of the present invention, the term "cnidocyst" will be used to refer both to cnidarian cnidocysts and myxozoan polar capsules.

The term "plant extracts" is used herein in the conventional sense to refer to concentrated preparations of plants obtained by removing the active constituents from the plant with suitable means. Such actives can be obtained from various parts of a plant such as seeds, needles, leaves, roots, bark, cones, stems, rhizomes, callus cells, protoplasts, organs and organ systems, and meristems. Suitable means for removal of the active ingredients include, for example, use of organic solvents, microwave or supercritical fluids extraction. Active ingredients are sometimes directly incorporated in food, pharmaceutical or cosmetical compositions in a variety of forms, including a pure or semi-pure component, a solid or liquid extract, or a solid plant matter. More often the plant extract is formulated into suitable preparations: compressed as tablets, made into pills, used to make infusions (teas), tinctures, etc., or mixed with excipients to make lotions, ointments, creams, etc. Non-limiting examples of plant extracts of the invention are tea extract, oat extract, thyme extract, olive leaf extract, wheat extract, liquorice extract, aloe barbadensis, chamomilla extract, lavandula extract, hamamelis extract, gingseng extract, hypericum extract, coffe extract, bamboo extract and koji extract. These and many other plant extracts are available in the market or can be obtained directly from the plant to be included in the compositions of the invention.

Plant extracts contain not only one but multiple contituents, many of them active. Often, the beneficial effect is derived from the combination of many of these active compounds, even though in some cases there is one particular compound that is mainly responsible for most of the activity.

The plant extract as used herein also includes "synthetic" extracts, i.e. various combinations of known plant components and/or constituents that are combined to substantially mimic the composition and/or activity of a plant extract of natural origin. The synthetic extracts will have two or more, three or more, or four or more active ingredients in common with a plant. Most preferably, the synthetic extracts will have substantially the same number of active ingredients. Natural or synthetic plant extracts that are enriched in one or several components are also considered part of the present invention.

As mentioned above, it is provided the use of compositions comprising plant extracts that inhibit the discharge of cnidocysts. These compositions are useful for avoiding the sting of poisonous organisms that contain cnidocysts such as cnidarians and myxozoans. Thus, in a particular embodiment of the first aspect of the invention, the cnidocyst to be inhibited belongs to an organism selected from the group consisting of Cnidaria and Myxozoa. Preferably, the compositions of the invention are used to avoid the sting of jellyfish.

As explained below, the inhibition of cnidocyst discharge by plant extracts has been evaluated by physical contact with gelatin probes than incorporate these extracts. It has also been found that phenolic compounds which are present in high concentrations in plant extracts, such as polyphenols or terpens, are highly effective in the inhibition of the discharge of cnidocysts. Further non-phenolic plant components like glycyrrhizate (which is a sugar compound) exhibit an inhibitory activity. It seems highly probable that the combination of various inhibitory compounds renders the plant extract highly effective in inhibiting cnidocyst discharge. In other words, in the presence of the plant extracts, the cnidocytes of the cnidarian organism, even though contacting with a foreign body, would not recognise it as danger or nourishment, with the result that its stinging organells would not be triggered.

In one embodiment, the plant extract to be included in the compositions of the invention is selected from those that contain phenolic compounds. In another embodiment, the plant extract to be included in the compositions of the invention is selected from those that contain glycyrrhizate, hydroxytyrosol and/or polyphenols. In a preferred embodiment of the invention the plant extract is selected from the group consisting of tea extract, oat extract, liquorice extract and olive leaf extract.The compositions of the invention can comprise one or more of these extracts.

An effective amount of tea extract, oat extract, liquorice extract or olive leaf extract is comprised between 0.001 and 10 % of the total composition. In one embodiment the effective amount of tea extract, oat extract, liquorice extract or olive leaf extract is comprised between 0.001 and 8 % of the total composition. In another embodiment, the effective amount of of tea extract, oat extract, liquorice extract or olive leaf extract is between 0.001 and 6 % of the total composition. In another embodiment, the effective amount of tea extract, oat extract, liquorice extract or olive leaf extract is between 0.001 and 5 %, preferably between 0.003 and 3 %, more preferably between 0.01 and 2 %, even more preferably between 0.1 and 2 % of the total composition. Concentrations are expressed in percentage by weight (% w/w) of active agents in the total composition.

The topical compositions of the invention are formulated in such a way as to provide a physical barrier over the skin that prevents that natural skin stimuli reach the cnidarian to trigger the mechanism of cnidocyst discharge. As well as exerting a barrier effect, the present compositioins contain an effective amount of one or more plant extracts as active agents that inhibit cnidocyst discharge. The inhibitory activity of the plant extract, together with the barrier effect of the topical formulation, is greatly effective in avoiding the sting of cnidarians.

The topical composition used is formulated preferably as an emulsion. An emulsion is a dispersed system comprising at least two immiscible phases, one phase dispersed in the other as droplets. An emulsifying agent is typically included to improve stability. When water is the dispersed phase and an oil is the dispersion medium, the emulsion is termed a water-in-oil emulsion (w/o). When an oil is dispersed as droplets throughout the aqueous phase, the emulsion is termed an oil-in-water emulsion (o/w). Other types of emulsions known in the art are multiple emulsions, such as water-in-oil-in-water emulsions (w/o/w), GELTRAP emulsions, where the aqueous intern phase is gelified and it is covered by the oil phase, and SWOP emulsions, also known as inversion emulsions. The emulsions for use in the sense of the present invention are preferably compatible with the following galenic formulations: natural sprays, aerosols, gels, creams, lotions and/or sticks. Other useful galenic formulations for topical administration include, but are not limmited to, ointments, liposomes, liquid suspensions, foams, or mixtures thereof. The topical composition can also be disposed on a sheet to form a wet wipe product. The emulsion to be selected depends upon the desired formulation. For instance, both water-in-oil and oil-in-water emulsions are compatible with natural spray, aerosol, lotion, cream, gel and stick formulations, while w/o/w emulsions, GELTRAP emulsions and SWOP emulsions are compatible with natural spray, aerosol, lotion, cream or gel formulations but not with a stick formulation. Further, the formulations used are preferably water resistant.

Topical formulations can be prepared according to methods well known in the state of the art. The appropriate carriers, and amounts thereof, can readily be determined by those skilled in the art according to the type of formulation being prepared. The components that constitute the base of the emulsion make up the "emulsifying system", to which the aqueous phase, the oil phase, active agents and further excipients are to be added. Non-limiting examples of emulsifying systems that are suitable for use in the present invention are disclosed in the examples below.

The compositions of the invention can contain one or more sunscreen agents. The sunscreen agents that can be used in the present invention must be capable of absorbing or blocking the harmful effects of ultraviolet radiation. In addition, they must be non-toxic and non-irritating when applied to the skin. The sunscreen agent can be included in the formulation at about 1 % to about 40 % (w/w) of the total composition. The amount of sunscreen agent in the composition will vary in the above range dependent upon the sun protection factor (SPF) desired. The higher the SPF, the greater the total amount of sunscreen agent. Preferably, the sunscreen agents are included at a concentration between 4 and 35 % (w/w) to achieve a SPF of 2 to 50+. More preferably, the sunscreen agents are included at a concentration between 10 and 30 %. Still more preferably, the sunscreen agents are included at a concentration between 19 and 23 % for high SPF products.

In one particular embodiment, the composition of the invention is a lotion that contains an emulsifying system that comprises polyglyceryl-4 diisostearate/polyhydroxystearate/sebacate, hydrogenated castor oil, and microcrystalline wax, and a sunscreen agent selected from the group consisting of octocrylene, butyl methoxydibenzoylmethane, bis-ethylhexyloxyphenol methoxyphenyl triazine, diethylhexyl butamido triazone, ethylhexyl triazone and titanium dioxide, or mixtures thereof.

The topical compositions defined above comprise appropriate excipients for topical administration that can be pharmaceutical and/or cosmetical excipients, including, but not limited to, emulsifiers, protectives, adsorbents, emollients, surfactants, stabilizers, preservatives, moisturizers, buffering agents, solubilizing agents, dry-feel agents, waterproofing agents, antifoaming agents and skin-penetration agents. The excipients used have affinity for the skin, are well tolerated, stable and are used in an amount adequate to provide the desired consistency and ease of application. Additionally, the compositions may contain other ingredients, such as fragances, colorants, and other components known in the state of the art for use in topical formulations.

The topical compositions of the invention can also incorporate additional active ingredients. Non-limiting examples of adequate active agents for use in the compositions of this invention are: skin-soothing agents, antihistamines, anti-oxidants, and vitamins.

In one embodiment, the additional active ingredient is an inhibitor of cnidocysts discharge. Compounds that have been previously described to be active against cnidocyst discharge are antihistamines and certain metal cations. Adequate metal cations in the sense of this invention are lanthanum, gadolinium, zinc, calcium, sodium, potassium, magnesium and titanium. The cation can be in the form of any suitable source, such as a salt, for example, a chloride salt. Preferably, the metal cation included in the composition of the invention is lanthanum.

In a preferred embodiment, the composition of the invention additionally comprises an external source of lanthanum cation in a concentration between 0.01 and 4 %, preferably 0.1 and 2 %, more preferably around 0.44 % of the total composition.

As mentioned above, the present inventors have found that the combination of plant extracts with metal cations have a synergistic effect in the inhibition of cnidocyst discharge.

The interaction between active agents can be studied by graphical means to find out whether the combination of two specific active components yields an additive, synergistic or antagonistic effect. The interaction analysis of the combinations of oat extract and green tea extract with lanthanum is illustrated in the Examples below.

The ratio to be used for the inhibitors of cnidocyst discharge that are included in the compositions of the invention can be between 1:500 and 1:1 (plant extract: metal cation). In a particular embodiment the ratio is between 1:400 and 1:2, preferably between 1:300 and 1:2, more preferably between 1:200 and 1:2, and even more preferably between 1:200 and 1:3 (plant extract: metal cation). In another embodiment the ratio is between 1:100 and 1:3, preferably between 1:50 and 1:3, more preferably between 1:25 and 1:3, and even more preferably between 1:10 and 1:4 (plant extract: metal cation).

In a preferred embodiment, the composition of the invention contains oat extract, tea extract, or a mixture thereof, and La³⁺. Preferably, the amount of La³⁺ is between 0.01 and 4 % of the total composition, the amount of oat extract is between 0.001 and 10 % of the total composition and the amount of tea extract is between 0.001 and 10 % of the total composition More preferably, the amount of La³⁺ is between 0.1 and 2 % of the total composition, the amount of oat extract is between 0.01 and 2 % of the total composition and the amount of tea extract is between 0.01 and 2 % of the total composition

Throughout the description and claims the word "comprise" and variations of the word, are not intended to exclude other technical features, additives, components, or steps. Unless otherwise defined, all technical and scientific terms used herein have the same meaning as commonly understood by one of ordinary skill in the art to which this invention belongs. Methods and materials similar or equivalent to those described herein can be used in the practice of the present invention.

Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### BRIEF DESCRIPTION OF THE DRAWINGS

FIG. 1. Oat extract and lanthanum interaction in the inhibition of cnidocyst discharge in Pelagia noctiluca. Number of discharged cnidocysts/mm² (Y). Oat extract 0.075% (A), oat extract 0.055% and La³⁺ 0.09% (B), oat extract 0.038% and La³⁺ 0.18% (C), oat extract 0.019% and La³⁺0.27% (D), La³⁺ 0.36% (E). Percentage concentrations expressed in w/v. Large cnidocysts (L), medium cnidocysts (M), small cnidocysts (S), total cnidocysts (T). Additivity line (α). Synergy curve (∅).
FIG. 2. Green tea extract and lanthanum interaction in the inhibition of cnidocyst discharge in Pelagia noctiluca. Number of discharged cnidocysts/mm² (Y). Tea extract 0.015% (A), tea extract 0.011 % and La³⁺ 0.03% (B), tea extract 0.008% and La³⁺ 0.06% (C), tea extract 0.004% and La³⁺ 0.09% (D), La³⁺ 0.12% (E). Percentage concentrations expressed in w/v. Large cnidocysts (L), medium cnidocysts (M), total cnidocysts (T). Additivity line (α). Synergy curve (∅).
FIG. 3. Pain scores at 0, 15, 30, 60, 90 and 120 minutes after completion of the tentacle application. Commercial lotion (1), test lotion (2), control (3).
FIG. 4. Clinical scores at 0, 15, 30, 60, 90 and 120 minutes after completion of the tentacle application. Commercial lotion (1),test lotion (2), control (3).
FIG. 5. Pieces of umbrella-proximal area of each tentacle in contact with four test zones on the forearms.

### DETAILED DESCRIPTION OF PARTICULAR EMBODIMENTS

### Example 1: emulsifying systems

The following emulsifying systems are useful when formulating the compositions of the invention.

**Table 1. Emulsifying systems for oil-in-water emulsion (o/w)**

| Non-ionic, anionic emulsions | |
|---|---|
| | % of total formulation (w/w) |
| Glyceryl stearate, PEG 100 stearate | 1.5-5 |
| Peg-40 stearate | 0.5-4 |
| Cetearyl alcohol | 0.5-4 |
| Sclerotium gum | 0.1-2 |
| Polyacrylamide, c13-14 isoparaffin | 0.1-2 |

| Cationic emulsions | |
|---|---|
| | % of total formulation (w/w) |
| Distearydimonium chloride | 2-6 |
| Dihydrogenated palmoylethyl hydroxyethylmonium | |
| methosulfate | 1-4 |
| Stearyl alcohol | 0-5 |
| Cetearyl alcohol | 0-5 |
| Glyceryl stearate | 0-3 |
| Polyacrylate-13 & polyisobutene & polysorbate 20 | 0.1-2 |

**Table 2. Emulsifying systems for water-in-oil emulsions (w/o)**

| | % of total formulation (w/w) |
|---|---|
| Polyglyceryl-4 isostearate | 1.5-5 |
| Microcristalline wax | 0.1-1 |
| Hydrogenated castor oil | 0.1-1 |

**Table 3. Emulsifying systems for multiple emulsions (w/o/w)**

| | % of total formulation (w/w) |
|---|---|
| Polyglyceryl-4 isostearate | 1.5-5 |
| Microcristalline wax | 0.1-1 |
| Hydrogenated castor oil | 0.1-1 |
| Acrylates (pemulen tr-2) | 0.05-2 |
| Polyacrylate-13 & polyisobutene & polysorbate 20 | 0.1-5 |

**Table 4. GELTRAP emulsions**

| | % of total formulation (w/w) |
|---|---|
| Octyldodecanol/octyldodecyl | 1.5-5 |
| Xyloside/peg-30 dipolyhydroxystearate | 1-6 |
| Polyacrylate-13 & polyisobutene & polysorbate 20 | 0.1-5 |

**Table 5. SWOP emulsions**

| Anionic emulsions | |
|---|---|
| | % of total formulation (w/w) |
| Polyglyceryl-4 isostearate | 1.5-5 |
| Sodium lauryl glucose carboxylate (20%) | 0.5-3 |
| Xanthan gum | 0.2-2 |
| Acrylamide/sodium acrylate copolymer; paraffinum | |
| liquidum; trideceth-6 | 0.5-2.5 |
| Myristyl myristate | 0.5-3 |

| Non-ionic emulsions | |
|---|---|
| | % of total formulation (w/w) |
| Polyglyceryl-4 isostearate | 1.5-5 |
| Decyl glucoside | 0.5-3 |
| Xanthan gum | 0.2-2 |
| Acrylamide/sodium acrylate copolymer; paraffinum | |
| liquidum; trideceth-6 | 0.5-2.5 |
| Myristyl myristate | 0.5-3 |

### Example 2: in vitro inhibition of cnidocyst discharge by plant extracts and phytocompounds

The efficacy of plant extracts and phytocompounds, in inhibiting cnidocyst discharge of Pelagia noctiluca (which is one of the most common species along the Mediterranean Sea) was evaluated by physical contact with a gelatin probe. Said probes, besides stimulating the cnidocyte battery, retained the different types of discharged cnidocysts that can have different sizes, thereby allowing a quantitative evaluation of the response. The results shown below demonstrate a strong inhibitory effect of several selected plant extracts and phytocompounds, in cnidocyst discharge.

Each gelatin probe was made coating one end of a 6 cm length of monofilament fishing line (1 mm diameter) with 25% (w/v) gelatin in deionized water and then placed in a humidified atmosphere during 30 minutes for gelatin solidification. The different substances to be tested were dissolved in deionized water at selected concentrations before the addition of gelatin: oat extract (Burgundi Cosmetics) and tea extract (Symrise lberica) were used at a final concentration of active agent of 0.025 % (w/v); dipotassium glycyrrhizate and hydroxytyrosol were used at a concentration of 0.5 M. Plain gelatin covered probes (no embebed active compound) were used as controls and represented maximum discharge figures.

After solidification, each gelatin probe was gently wiped across oral arms of Pelagia noctiluca 5 times from 1 to 5 seconds in the tanks. The probes were then placed in humidified atmosphere plates and observed under a steroemicroscope for cnidocyst quantification. Individual cnidocysts that had discharged into the gelatin probes were counted in 7 different fields of view and the mean value was calculated. Results are the average of triplicate probes.

An inhibitory effect was considered if the substance diminished the number of discharged nematocytes per mm² of gelatin probe as compared with controls.

The effect of oat extract, green tea extract, dipotassium glycyrrhizate and hydroxytirosol in cnidocyst discharge is shown in table 6 expressed in % of discharged cnidocysts/mm² and the corresponding percentage of inhibition. Equivalent results were found for the effect of these agents on the inhibition of cnidocyst discharge in Rhizostoma pulmo, which is also one of the most common jellyfish species along the Mediterranean (results not shown).

**Table 6. Effect of plant extracts and derivatives thereof in cnidocyst discharge**

| Discharged cnidocysts/mm² (%) | | Inhibition (%) |
|---|---|---|
| Control | 100 | 0 |
| Oat extract | 47,33 | -52,67 |
| Green tea extract | 46,25 | -53,75 |
| Dipotassium glycyrrhizate | 32,7 | -67,3 |
| WHidroxytyrosol | 43,6 | -56,4 |

### Example 3: combination of cnidocyst discharge inhibitors

Two substances that produce similar effects will sometimes produce exaggerated (synergism), diminished (antagonism) or similar (additivity) effects when used concurrently. A quantitative assessment is necessary to distinguish the synergism and the antagonism cases from a simply additive action.

Although no uniformity about the calculations and definitions of interactions exists, the most accepted description defines an interaction when the observed effect of the combination differs with the effects of the individual constituents. The interaction studies of two substances involve both, the individual efficacy evaluation and the effects of different combinations.

The graphical representation of the interaction between two active compounds could be expressed as follows. The x-axis shows the dose pair's combinations at the individual substance concentrations tested in the study, and in the y-axis the results obtained expressed as number of discharged nematocysts per mm². The straight line connecting the effects of both substances on their own is the locus of points that will produce this effect in a simple additive combination, the additivity line. A result below the line of additivity suggests a synergism and a result over the line an antagonism.

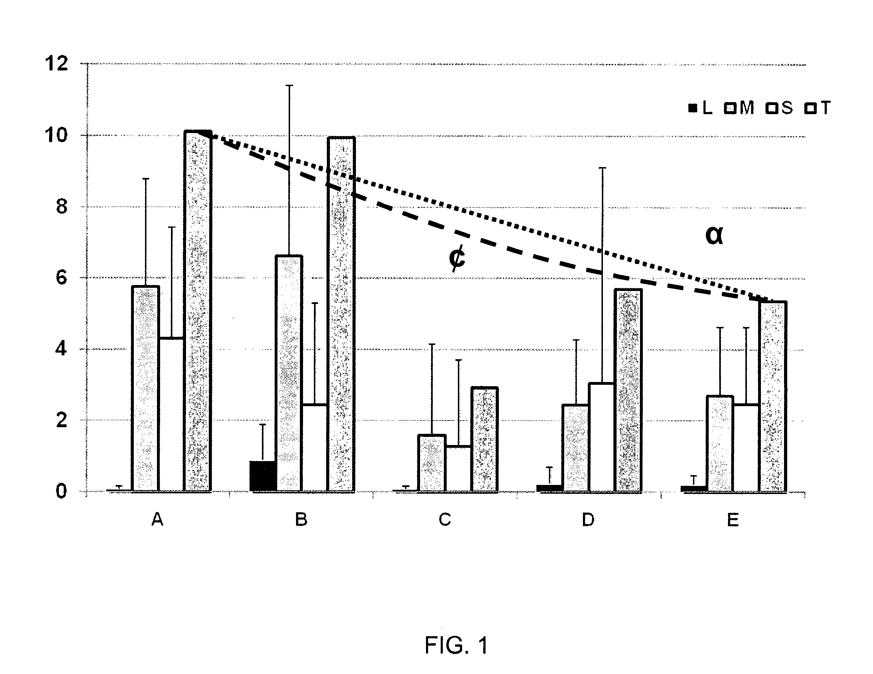

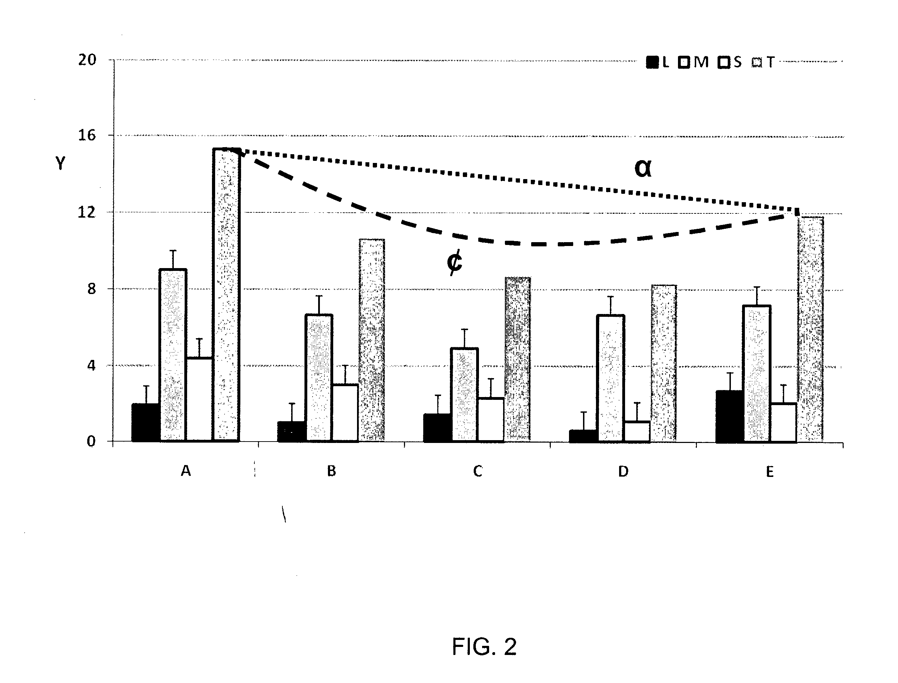

FIG. 1 and FIG. 2 represent, respectively, the interaction between oat extract and lanthanum and tea extract and lanthanum in the inhibition of cnidocyst discharge. The number of discharged cnidocysts in Pelagia noctiluca was analysed for each combination of active agents by physical contact with a gelatin probe as described in Example 2. The doses of each of the constituents are referred as concentrations of active agent, i.e. of pure oat or green tea extract, and lanthanum cation. In preliminar experiments, the employed doses for the individual constituents had been found to be effective in inhibiting cnidocyst discharge. The results show that the combinations are more effective than each component on its own, thus exhibiting a synergistic interaction.

### Example 4: in vivo inhibition of cnidocyst discharge in Pelagia noctiluca

In addition to the in vitro experiments disclosed above, the ability of a composition according to the invention to avoid the sting of Pelagia noctiluca was evaluated in vivo and compared with a commercial anti-jellyfish lotion.

The composition of the invention was formulated as a lotion and comprised the following ingredients:

| Test lotion | % (w/w) |
|---|---|
| Aqua (water) | 51,161 |
| Polyglyceryl-4 | |
| diisostearate/polyhydroxystearate/sebacate | 2,000 |
| Lanthanum chloride | 1,200 |
| Avena sativa (oat) kernel extract | 0,005 |
| Camellia sinensis leaf extract | 0,003 |
| Hygrogenated castor oil | 0,100 |
| Microcrystalline wax | 0,100 |

Twenty one subjects with no obvious skin disease or known dermatological pathology were included in the study. Subjects were instructed not to use moisturisers or any care product on the volar forearms during three days before.

After delimitation of 4 zones, 2 investigational products zones for the formulation of the invention, 1 commercial formulation zone and 1 untreated control zone, forming each a square (6 cm x 4 cm) on the inner forearms, the products (the test lotion comprising the composition of the invention and the commercial lotion) were applied according to a randomization protocol and rubbed into the skin with a fingerstall on the basis of 2mg/cm²(50µl on 28cm²) per zone. The products were allowed to dry and penetrate for 30 minutes.

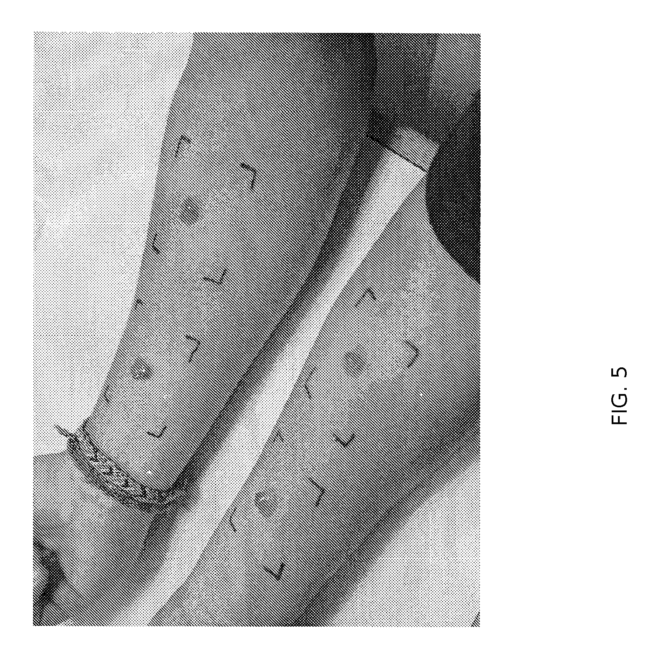

Tentacles were removed from live and well-being Pelagia noctiluca jellyfish in tanks with sea water. For each volunteer 4 tentacles of the same jellyfish were used. Equivalent size pieces of umbrella-proximal area of each tentacle were left in contact with the four zones on the forearms for 3 minutes (for ilustration see figure 5).

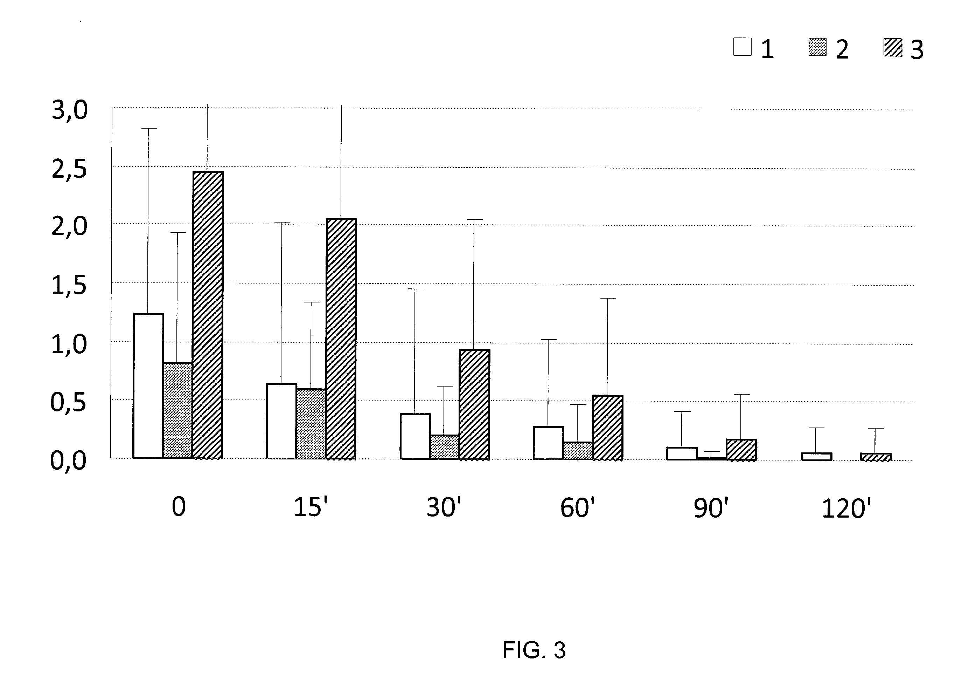

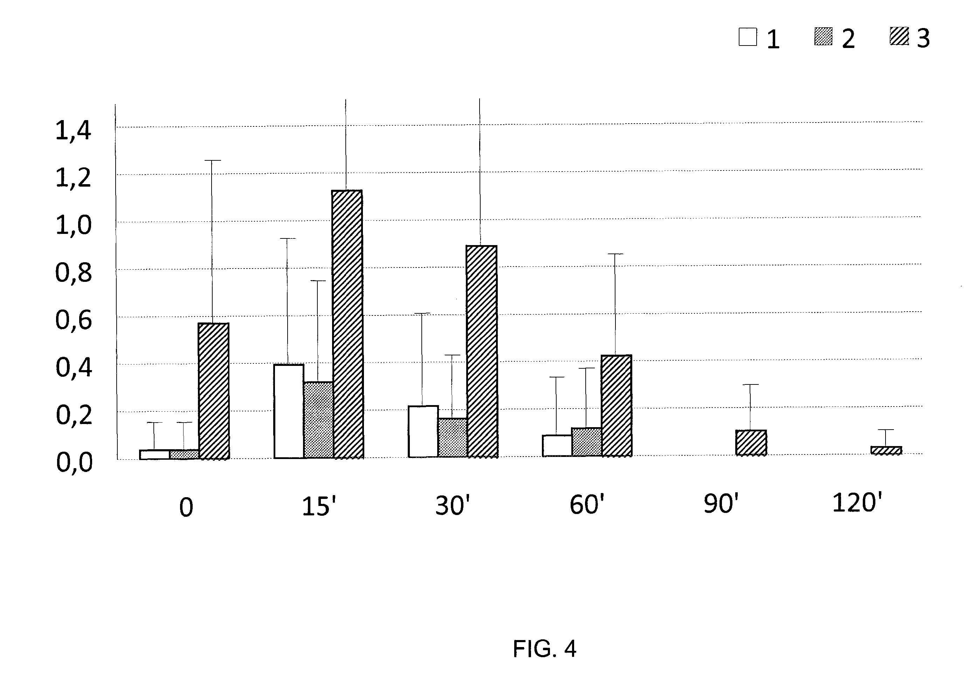

After removal of the tentacles, subjects were examined by a dermatologist and queried about pain. The clinical score measures the degree of inflammation in a 0-3 scale according to the following criteria: 0 (no change), 1 (erythema), 2 (oedema), 3 (papules). Pain was subjectively scored by the volunteer on a 0 (no discomfort) to 5 (maximum pain) scale. Figures 3 and 4 respectively represent the results obtained for pain and clinical scores at 0, 15, 30, 60, 90 and 120 minutes after completion of the tentacle application.

The resuts demonstrate the ability of the compositions of the invention to avoid the stinging of jellyfish in vivo as measured by the reduction of pain and clinical symthoms. Further, the composition of the invention scores better than the commercial lotion at the measured parameters.

### REFERENCES CITED IN THE DESCRIPTION

- Salleo et al., J. exp. Biol., 1994, vol. 187, p. 201-206
- WO 98/53807
- WO 05/076731
- Boer Kimball A. et al, Wilderness and Environmental Medicine, 2004, vol. 15, p. 102-108

## Claims

1. Use of a topical composition comprising an effective amount of at least one plant extract, together with appropriate topical pharmaceutically and/or cosmetically acceptable carriers and/or excipients, for avoiding the sting of poisonous organisms that contain cnidocysts, wherein the plant extract is selected from those that contain glycyrrhizate, hydroxytyrosol and/or polyphenols.

2. Use of the composition according to claim 1, wherein the plant extract is selected from the group consisting of tea extract, oat extract, liquorice extract, and olive leaf extract.

3. Use of the composition according to claim 2, wherein the amount of tea extract if present is comprised between 0.001 and 10 % (w/w) of the total composition, the amount of oat extract if present is comprised between 0.001 and 10 % (w/w) of the total composition, the amount of liquorice extract if present is comprised between 0.001 and 10 % (w/w) of the total composition, and the amount of olive leaf extract if present is comprised between 0.001 and 10 % (w/w) of the total composition.

4. Use of the composition according to claim 3, wherein the amount of tea extract if present is comprised between 0.003 and 3 % (w/w) of the total composition, the amount of oat extract if present is comprised between 0.003 and 3 % (w/w) of the total composition, the amount of liquorice extract if present is comprised between 0.003 and 3 % (w/w) of the total composition, and the amount of olive leaf extract if present is comprised between 0.003 and 3 % (w/w) of the total composition.

5. Use of the composition according to any of the claims 1-4, further comprising an emulsion system, and one or more cosmetically or pharmaceutically active agents selected from the group consisting of sunscreen agents, skin-soothing agents, antihistamines, anti-oxidants and vitamins.

6. Use of the composition according to claim 5, which is for administration in the form of a cream, a lotion, a natural spray, an aerosol, a gel, a foam, a suspension, a wipe, or a stick.

7. Use of the composition according to claims 5-6, wherein the emulsion system comprises polyglyceryl-4 diisostearate/polyhydroxystearate/sebacate, hydrogenated castor oil, and microcrystalline wax, and the sunscreen agent is selected from the group consisting of octocrylene, butyl methoxydibenzoylmethane, bis-ethylhexyloxyphenol methoxyphenyl triazine, diethylhexyl butamido triazone, ethylhexyl triazone, titanium dioxide, and mixtures thereof.

8. Use of the composition according to any of the claims 1-7, further comprising an external source of a metal cation selected from the group consisting of lanthanum, gadolinium, zinc, calcium, sodium, potassium, magnesium, and titanium.

9. Use of the composition according to claim 8, wherein the cation is La³⁺.

10. Use of the composition according to any of the claims 8-9, comprising La³⁺ in an amount between 0.01 and 4 % (w/w) of the total composition, and a plant extract selected from the group consisting of oat extract in an amount between 0.001 and 10 % (w/w) of the total composition and tea extract in an amount between 0.001 and 10 % (w/w) of the total composition.

11. Use of the composition according to any of the claims 1-10, wherein the cnidocyst is from an organism selected from the group consisting of Cnidaria and Myxozoa.

12. A method for avoiding the sting of an organism selected from the group consisting of Cnidaria and Myxozoa comprising the topical administration of the composition as defined in any of the claims 1-11, prior to contact with said organism.

13. Use of a combination of an effective amount of at least one plant extract selected from the group consisting of tea extract, oat extract, liquorice extract, and olive leaf extract and an effective amount of an external source of a metal cation selected from the group consisting of lanthanum, gadolinium, zinc, calcium, sodium, potassium, magnesium, and titanium for simultaneous or sequential administration for avoiding the sting of poisonous organisms that contain cnidocysts, wherein the amount of tea extract if present is comprised between 0.001 and 10 % (w/w) of the total composition, the amount of oat extract if present is comprised between 0.001 and 10 % (w/w) of the total composition, the amount of liquorice extract if present is comprised between 0.001 and 10 % (w/w) of the total composition, and the amount of olive leaf extract if present is comprised between 0.001 and 10 % (w/w) of the total composition.

14. Use of the combination according to claim 13, wherein the metal cation is lanthanum and is comprised in an amount between 0.1 and 4% of the total composition.

## Patentansprüche

1. Verwendung einer topischen Zusammensetzung umfassend eine wirksame Menge von mindestens einem Pflanzenextrakt nebst geeigneten topischen pharmazeutisch und/oder kosmetisch akzeptablen Trägerstoffen und/oder Hilfsstoffen zur Verhinderung des Stiches von giftigen Organismen, die Cnidozysten beinhalten, wobei der Pflanzenextrakt aus denen ausgewählt ist, die Glycyrrhizat, Hydroxytyrosol und/oder Polyphenolen enthalten.

2. Verwendung einer Zusammensetzung nach Anspruch 1, wobei der Pflanzenextrakt ausgewählt ist aus der Gruppe bestehend aus Tee-Extrakt, Haferextrakt, Lakritzextrakt, und Olivenbaumblätterextrakt.

3. Verwendung der Zusammensetzung nach Anspruch 2, wobei die Menge von Tee-Extrakt, wenn dieser vorhanden ist, bei zwischen 0,001 und 10 Gew.-% der gesammten Zusammensetzung liegt, die Menge von Haferextrakt, wenn dieser vorhanden ist, bei zwischen 0,001 und 10 Gew.-% der gesammten Zusammensetzung liegt, die Menge von Lakritzextrakt, wenn dieser vorhanden ist, bei zwischen 0,001 und 10 Gew.-% der gesammten Zusammensetzung liegt, und die Menge von Olivenbaumblätterextrakt, wenn dieser vorhanden ist, bei zwischen 0,001 und 10 Gew.-% der gesammten Zusammensetzung liegt.

4. Verwendung der Zusammensetzung nach Anspruch 3, wobei die Menge von Tee-Extrakt, wenn dieser vorhanden ist, bei zwischen 0,003 und 3 Gew.-% der gesammten Zusammensetzung liegt, die Menge von Haferextrakt, wenn dieser vorhanden ist, bei zwischen 0,003 und 3 Gew.-% der gesammten Zusammensetzung liegt, die Menge von Lakritzextrakt, wenn dieser vorhanden ist, bei zwischen 0,003 und 3 Gew.-% der gesammten Zusammensetzung liegt, und die Menge von Olivenbaumblätterextrakt, wenn dieser vorhanden ist, bei zwischen 0,003 und 3 Gew.-% der gesammten Zusammensetzung liegt.

5. Verwendung der Zusammensetzung nach einem der Ansprüche 1-4 weiterhin umfassend ein Emulsionssystem, und einen oder mehrere kosmetische oder pharmazeutische Wirkstoffe, die ausgewählt sind aus der Gruppe bestehend aus Sonnenschutzmitteln, hautberuhigenden Mitteln, Antihistaminika, Antioxidationsmitteln und Vitaminen.

6. Verwendung der Zusammensetzung nach Anspruch 5 zur Verabreichung in Form einer Creme, einer Lotion, eines natürlichen Sprays, eines Aerosols, eines Gels, eines Schaums, einer Aufschwemmung, eines Feuchttuchs, oder eines Stiftes.

7. Verwendung der Zusammensetzung nach Anprüchen 5-6, wobei das Emulsionssystem Polyglyceryl-4 Diisostearat/Polyhydroxystearat/Sebacat, hydriertes Rizinusöl und mikrokristallines Wachs umfasst, und das Sonnenschutzmittel ausgewählt ist aus der Gruppe bestehend aus Octocrylen, Butyl Methoxydibenzoyl-Methan, Bis-Ethylhexyloxyphenol Methoxyphenyl Triazin, Diethylhexyl Butamido Triazon, Ethylhexyl Triazon, Titaniumdioxid, und Mischungen davon.

8. Verwendung der Zusammensetzung nach einem der Ansprüche 1-7 weiterhin umfassend eine externe Quelle von einem metallischen Kation, das ausgewählt ist aus der Gruppe bestehend aus Lanthanum, Gadolinium, Zink, Calcium, Natrium, Kalium, Magnesium, und Titanium.

9. Verwendung der Zusammensetzung nach Anspruch 8, wobei das Kation La³⁺ ist.

10. Verwendung der Zusammensetzung nach einem der Ansprüche 8-9 umfassend La³⁺ in einer Menge, die bei zwischen 0,01 und 4 Gew.-% der gesammten Zusammensetzung liegt, und umfassend einen Pflanzenextrakt, der ausgewählt ist aus der Gruppe bestehend aus Haferextrakt in einer Menge, die bei zwischen 0,001 und 10 Gew.-% der gesammten Zusammensetzung liegt und Tee-Extrakt in einer Menge, die bei zwischen 0,001 und 10 Gew.-% der gesammten Zusammensetzung liegt.

11. Verwendung der Zusammensetzung nach einem der Ansprüche 1-10, wobei die Cnidozyste aus einem Organismus ist, der aus der Gruppe bestehend aus Cnidaria und Myxozoa ausgewählt ist.

12. Methode zur Verhinderung des Stiches von einem Organismus, der ausgewählt ist aus der Gruppe bestehend aus Cniadria und Myxozoa umfassend die topische Verabreichung der Zusammensetzung wie in einem der Ansprüche 1-11 definiert, bevor es zum Kontakt mit dem Organismus kommt.

13. Verwendung einer Kombination von einer wirksamen Menge von mindestens eines Pflanzenextraktes, der ausgewählt ist aus der Gruppe bestehend aus Tee-Extrakt, Haferextrakt, Lakritzextrakt, und Olivenbaumblätterextrakt und einer wirksamen Menge von einer externen Quelle von einem metallischen Kation, das ausgewählt ist aus der Gruppe bestehend aus Lanthanum, Gadolinium, Zink, Calcium, Natrium, Kalium, Magnesium, und Titanium zur gleichzeitigen oder sequentiellen Verabreichung zur Verhinderung des Stiches von giftigen Organismen, die Cnidozysten beinhalten, wobei die Menge von Tee-Extrakt, wenn dieser vorhanden ist, bei zwischen 0,001 und 10 Gew.-% der gesammten Zusammensetzung liegt, die Menge von Haferextrakt, wenn dieser vorhanden ist, bei zwischen 0,001 und 10 Gew.-% der gesammten Zusammensetzung liegt, die Menge von Lakritzextrakt, wenn dieser vorhanden ist, bei zwischen 0,001 und 10 Gew.-% der gesammten Zusammensetzung liegt, und die Menge von Olivenbaumblätterextrakt, wenn dieser vorhanden ist, bei 0,001 und 10 Gew.-% der gesammten Zusammensetzung liegt.

14. Verwendung der Zusammensetzung nach Anspruch 13, wobei das metallische Kation Lanthanum ist und dessen Menge bei zwischen 0,1 und 4 Gew.-% der gesammten Zusammensetzung liegt.

## Revendications

1. Utilisation d'une composition topique comprenant une quantité effective d'au moins un extrait de plante avec des véhicules et/ou des excipients topiques appropriés pharmaceutiquement et/ou cosmétiquement acceptables, pour éviter la piqûre d'organismes venimeux qui contiennent des cnidocystes, dans laquelle l'extrait de plante est choisi dans ceux qui contiennent du glycyrrhizate, de l'hydroxytyrosol et/ou des polyphénols.

2. Utilisation de la composition selon la revendication 1, dans laquelle l'extrait de plante est choisi dans le groupe constitué d'extrait de thé, d'extrait d'avoine, d'extrait de réglisse, et d'extrait de feuille d'olivier.

3. Utilisation de la composition selon la revendication 2, dans laquelle la quantité d'extrait de thé, lorsqu'il est présent, est comprise entre 0,001 et 10 % en poids de la composition totale, la quantité d'extrait d'avoine, lorsqu'il est présent, est comprise entre 0,001 et 10% en poids de la composition totale, la quantité d'extrait de réglisse, lorsqu'il est présent, est comprise entre 0,001 et 10% en poids de la composition totale, et la quantité d'extrait de feuille d'olivier, lorsqu'il est présent, est comprise entre 0,001 et 10% en poids de la composition totale.

4. Utilisation de la composition selon la revendication 3, dans laquelle la quantité d'extrait de thé, lorsqu'il est présent, est comprise entre 0,003 et 3 % en poids de la composition totale, la quantité d'extrait d'avoine, lorsqu'il est présent, est comprise entre 0,003 et 3 % en poids de la composition totale, la quantité d'extrait de réglisse, lorsqu'il est présent, est comprise entre 0,003 et 3 % en poids de la composition totale, et la quantité d'extrait de feuille d'olivier, lorsqu'il est présent, est comprise entre 0,003 et 3 % en poids de la composition totale.

5. Utilisation de la composition selon l'une quelconque des revendications 1-4 comprenant en outre un système d'émulsion, et un ou plusieurs agents cosmétiquement ou pharmaceutiquements actifs choisis dans le groupe constitué des écrans solaires, des agents apaisants de la peau, des antihistaminiques, des antioxydants et des vitamines.

6. Utilisation selon la revendication 5, pour l'administration sous la forme d'une crème, d'une lotion, d'un spray naturel, d'un aérosol, d'un gel, d'une mousse, d'une suspension, d'une lingette, ou d'un stick.

7. Utilisation de la composition selon les revendications 5-6, dans laquelle le système d'émulsion comprend du polyglycéryl-4 diisostéarate/polyhydroxystéarate/sébacate, de l'huile de ricin hydrogéné, et de la cire microcristalline, et l'écran solaire est choisi dans le groupe constitué de l'octocrylène, du butyl méthoxydibenzoyl-méthane, de la bis-éthylhéxyloxyphénol méthoxyphényl triazine, de la diéthylhéxyl butamido triazone, de la éthylhéxyl triazone, du dioxyde de titanium, et des mélanges de ceux-ci.

8. Utilisation de la composition selon l'une quelconque des revendications 1-7 comprenant en outre une source externe d'un cation métallique choisi dans le groupe constitué du lanthanum, du gadolinium, du zinc, du calcium, du sodium, du potassium, du magnésium, et du titanium.

9. Utilisation de la composition selon la revendication 8, dans laquelle le cation est le La³⁺.

10. Utilisation de la composition selon l'une quelconque des revendications 8-9 comprenant du La³⁺ dans une quantité d'entre 0,01 et 4% en poids de la composition totale, et un extrait de plante choisi du groupe constitué de l'extrait d'avoine dans une quantité d'entre 0,001 et 10% en poids de la composition totale et de l'extrait de thé dans une quantité d'entre 0,001 et 10% en poids de la composition totale.

11. Utilisation de la composition selon l'une quelconque des revendications 1-10, dans laquelle le cnidocyste est d'un organisme choisi dans le groupe constitué de Cnidaria et Myxozoa.

12. Méthode pour éviter la piqûre d'un organisme choisi dans le groupe constitué de Cnidaria et Myxozoa comprenant l'administration topique de la composition telle que définie dans l'une quelconque des revendications 1-11 avant le contact avec ledit organisme.

13. Utilisation d'une combinaison d'une quantité efficace d'au moins un extrait de plante choisi dans le groupe constitué de l'extrait de thé, de l'extrait d'avoine, de l'extrait de réglisse, et de l'extrait de feuille d'olivier et une quantité efficace d'une source externe d'un cation métallique choisi dans le groupe constitué du lanthanum, du gadolinium, du zinc, du calcium, du sodium, du potassium, du magnésium, et du titanium pour l'administration simultanée ou séquentielle pour éviter la piqûre d'organismes venimeux qui contiennent des cnidocystes, dans laquelle la quantité d'extrait de thé, lorsqu'il est présent, est comprise entre 0,001 et 10% en poids de la composition totale, la quantité d'extrait d'avoine, lorsqu'il est présent, est comprise entre 0,001 et 10% en poids de la composition totale, la quantité d'extrait de réglisse, lorsqu'il est présent, est comprise entre 0,001 et 10% en poids de la composition totale, et la quantité d'extrait de feuille d'olivier, lorsqu'il est présent, est comprise entre 0,001 et 10% en poids de la composition totale.

14. Utilisation de la combinaison selon la revendication 13, dans laquelle le cation métallique est le lanthanum et il est compris dans une quantité d'entre 0,1 et 4% de la composition totale.
